# EUROPÄISCHE PATENTSCHRIFT

(11) **EP 1 710 208 B1**
(45) Veröffentlichungstag und Bekanntmachung des Hinweises auf die Patenterteilung: **26.11.2008**
(21) Anmeldenummer: 05007378.2
(22) Anmeldetag: 05.04.2005
(51) Int. Cl.: C01G 9/00, C09C 1/04, A61K 33/30

(54) **Schwefel-modifiziertes Zinkoxid der Formel Zn(1-y)M(y)O(1-x)S(x) (x=0.01-0.08, y=0-0,2)**
Sulfur-modified Zink oxide of the formula Zn(1-y)M(y)O(1-x)S(x) (x=0.01-0.08, y=0-0,2)
Oxyde de zinc modifié avec du soufre correspondant à la formule Zn(1-y)M(y)O(1-x)S(x) (x=0.01-0.08, y=0-0,2)

(43) Veröffentlichungstag der Anmeldung: 11.10.2006
(73) Patentinhaber: Universität Hannover, 30167 Hannover (DE)
(72) Erfinder: Binnewies, Michael, 48161 Münster (DE); Locmelis, Sonja, Dr., 30455 Hannover (DE)
(74) Vertreter: Eisenführ, Speiser & Partner

(56) Entgegenhaltungen:
- WO-A-20/05024612
- Y.-Z. YOO, ZHENG-WU JIN, T. CHIKYOW, T. FUKUMURA, M. KAWASAKI, H. KOINUMA: "S doping in ZnO-Films by supplying ZnS-species with pulsed-laser-deposition method" APPLIED PHYSICS LETTERS, Bd. 81, Nr. 20, 11. November 2002 (2002-11-11), Seiten 3798-3400, XP002373920
- B.K. MEYER, A. POLITY, B. FARANGIS, Y. HE, D. HASSELKAMP, TH. KRÄMER, C. WANG: "Structural properties and bandgap bowing ZnO(1-x)Sx thin films deposited by reactive sputtering" APPLIED PHYSICS LETTERS, Bd. 85, Nr. 21, 22. November 2004 (2004-11-22), Seiten 4929-4931, XP002373921

## Beschreibung

Die vorliegende Erfindung betrifft ein modifiziertes Zinkoxid, das in Form von Partikeln oder als Pulver vorliegt, d. h. als bulk-Material. Die Partikel können dabei eine beträchtliche Größe besitzen, die mehrere Millimeter betragen oder sogar überschreiten kann.

Die Erfindung betrifft femer Artikel, welche erfindungsgemäße Partikel oder Pulver umfassen, die Verwendung eines erfindungsgemäßen Partikels oder Pulvers als UV-Absorptionsmittel sowie Verfahren zur Herstellung der erfindungsgemäßen Partikel oder Pulver.

Die Verwendung anorganischer Sauerstoffverbindungen wie zum Beispiel Titan(IV)-oxid und Zinkoxid als Absorber von ultraviolettem Licht, zum Beispiel in kosmetischen Sonnenschutzmüteln oder in Kunststoffen, Farben und Lacken, ist seit langem bekannt. Zinkoxid wird hierbei in besonders starkem Maße eingesetzt, da es ― anders als Titan(IV)-oxid ― keine photokatalytischen Eigenschaften besitzt.

Nachteiligerweise liegt jedoch die Absorptionskante des Zinkoxids bei ca. 380 nm, so dass eine zufriedenstellende Absorption im längerwelligen UVA-Bereich nicht möglich ist.

Es war daher die primäre Aufgabe der vorliegenden Erfindung, ein anorganisches UV-Absorptionsmittel anzugeben, welches auch im längerwelligen UVA-Bereich ein starkes Absvrptionsverhalten aufweist.

Erfindungsgemäß wird diese Aufgabe gelöst durch Angabe von Partikeln oder Pulvern einer Verbindung der Formel Zn_{1-y}M_{y}O₁₋ₓSₓ, in der x einen Wert im Bereich von 0,01 bis 0,08, M ein zweiwertiges Metall ausgewählt aus der Gruppe bestehend aus Cr, Mn, Fe, Co, Ni, Cu, Cd und Mg bedeutet und y einen Wert im Bereich von 0 bis 0,2 besitzt, wobei die Verbindung Wurtzit-Struktur besitzt.

Dünne Filme einer Verbindung der Formel ZnO₁₋ₓSₓ wurden bereits von B. K. Meyer et al. in den Applied Physics Letters, 85, 21, 4929. November 2004 veröffentlicht. Die genannte Veröffentlichung offenbart jedoch keine Partikel oder Pulver einer derartigen Verbindung, wie sie für einen Einsatz im industriellen Maßstab erforderlich wäre. Zudem offenbaren Meyer et al. lediglich Filme mit einer Dicke von ungefähr 300 nm; ein Hinweis, dass die Materialien thermodynamisch stabil und auch als bulk-Material präparativ erhälfilich sind, findet sich in der Veröffentlichung nicht. Entsprechendes gilt für die Veröffentlichung Y.-Z. Yoo, T.-W. Jin. T. Chikow, T. Fukurama, M. Kawasaki, H. Koinuma, Appl. Phys. Lett. 2002, 81, 3798.

Die Druckschrift WO2005024612 offenbart ebenfalls dünne optische Funktionsschichten, die aus ZnOₓS₍₁₋ₓ₎ (x=0-1) in kristalliner Form bestehen und die Wurtzit-Struktur aufweisen. Die Funktionsschichten werden hierbei auf Substrate unter Verwendung von bekannten Dünnschicht-Depositionsverfahren aufgebracht.

Überraschenderweise gelang es nun, erfindungsgemäße Partikel in Form hellgrünlicher, verwachsener Kristalle zu erhalten, die sich in ihrem Habitus deutlich von dem der nadelförmigen ZnO-Kristalle unterscheiden. Zudem konnten polykristalline, hellgelbe erfindungsgemäße Pulver erhalten werden.

Die erfindungsgemäßen Partikel oder Pulver einer Verbindung der Formel Zn_{1-y}M_{y}O₁₋ₓSₓ besitzen eine Absorptionskante, die im Vergleich mit der Basisverbindung ZnO verschoben ist. Das Ausmaß der Verschiebung der Absorptionskante und die Modifikation des sonstigen Absorptionsverhaltens hängen dabei von den Mengen des in die Wurlzit-Struktur eingebauten Schwefels und zweiwertigen Metalls ab. Für die Verbindung ZnO_{0.92}S_{0.08} ergibt sich eine Absorptionskante von ca. 430 nm, die im Vergleich mit der Absorptionskante von ca. 380 nm für ZnO also in den längerwelligen Bereich verschoben ist. Diese Verschiebung ist insbesondere deshalb überraschend, weil die Absorptionskante für ZnS ca. bei 350 nm liegt und die erfindungsgemäß einzusetzende Verbindung somit eine Absorptionskante besitzt, die nicht zwischen denen von ZnO und ZnS liegt. Diese Erkenntnis wird bestätigt durch die Zahlenwerte des Bandabstandes Eg: E_{g}(ZnO) = 3,19 eV, E_{g}(ZnO_{0,95}S_{0,05}) = 3,01 eV, E_{g}(ZnS) = 3,54 eV. Innerhalb des Bereiches von 380 nm bis 430 nm können für Verbindungen des Typs ZnO₁₋ₓSₓ unterschiedliche Absorptionskanten eingestellt werden, abhängig vom ausgewählten Wert für x.

Besonders überzeugende Ergebnisse ließen sich mit erfindungsgemäßen Partikeln oder Pulvern einer Verbindung der Formel Zn_{1-y}M_{y}O₁₋ₓSₓ erreichen, in der y den Wert 0 oder einen Wert im Bereich von 0-0,0002 besitzt. Solche erfindungsgemäßen Partikel oder Pulver lassen sich unter Verwendung von ZnO-Qualitäten mit einem entsprechend geringen Verunreinigungsgrad herstellen, siehe dazu unten. Insbesondere, aber nicht nur, wenn y den Wert 0 besitzt, also kein weiteres zweiwertiges Metall in die Verbindung eingebaut ist, besitzt x vorzugsweise einen Wert im Bereich von 0,01-0,05. In diesem Bereich ist die Verbindung bei 900 °C thermodynamisch stabil.

Die erfindungsgemäßen Partikel oder Pulver sind vorteilhafterweise einphasig, sie können aber auch mit anderen Partikeln bzw. Pulvern zu entsprechenden Mischungen kombiniert werden, z. B. mit Zinkoxid.

Bevorzugte erfindungsgemäße Partikel und Pulver besitzen eine Partikelgröße bzw. mittlere Komgröße von 300 nm oder mehr. Vorzugsweise liegt die Partikelgröße bzw. mittlere Korngröße im Bereich von 1-10 µm.

In den erfindungsgemäßen Partikeln oder Pulvern ist M vorzugsweise ausgewählt aus der Gruppe bestehend aus: Cr, Mn, Fe, Co, Ni, Cu, Cd und Mg.

Zur Herstellung entsprechender Partikel oder Pulver wird vorzugsweise zunächst eine Verbindung der Formel Zn_{1-y}M_{y}O hergestellt und diese anschließend in die Verbindung der Formel Zn_{1-y}MyO₁₋ₓSₓ überführt. Hinsichtlich der Herstellung von Verbindungen der Formel Zn_{1-y}M_{y}O wird insoweit auf die folgenden Veröffentlichungen verwiesen: S. Locmelis, R. Wartchow, G. Patzke, M. Binnewies; Z. Anorg, Allg. Chem. 1999, 625, 661; S. Locmelis, M. Binnewies; Z. Anorg. Allg. Chem. 1999, 625, 1573; S. Locmelis, M. Binnewies; Z. Anorg. Allg. Chem. 1999, 625, 1578; G. Patzke, S. Locmelis, R. Wartchow, M. Binnewies; J. Cryst. Growth 1999, 203, 141; K. Ullrich, S. Locmelis, M. Binnewies, K. D. Becker: Phase Transitions, 2003, 76, 103; S. Locmelis, Dissertation Universität Hannover 1999**;** B. K. Meyer, A. Polity, B. Farangis, Y. He, D. Hasselkamp, Th. Krämer, C. Wang, Appl. Phys. Lett. 2004, 85, 4929. Der Fachmann wird anhand weniger Vorversuche ermitteln, ob und gegebenenfalls welche Beschränkungen es hinsichtlich der in die Wurtzit-Struktur einbaubaren Mengen an Metall M und Schwefel gibt. Die vorstehend angegebenen Maximalwerte für x und y sind nicht für jedes Metall M wünschenswert und/oder erreichbar.

Die Erfindung betrifft auch Artikel, die erfindungsgemäße Partikel oder Pulver umfassen. Vorzugsweise umfassen erfindungsgemäße Artikel die Partikel oder das Pulver im Bereich ihrer Oberfläche, so dass die Partikel oder das Pulver bei Gebrauch des jeweiligen Artikels eine UV-absorbierende Wirkung entfalten können.

Bevorzugte erfindungsgemäße Artikel sind kosmetische Zubereitungen (insbesondere Sonnenschutzformuiierungen). Kunststoffe, Farben und Lacke. Jeder der genannten Artikel umfasst vorzugsweise eine solche Menge an erfindungsgemäßen Partikeln oder Pulver, dass eine UV-absorbierende Wirkung eintritt.

Die vorliegende Erfindung betrifft dementsprechend auch die Verwendung eines erfindungsgemäßen Partikels oder Pulvers als UV-Absorptionsmittel.

Die erfindungsgemäßen Partikel oder Pulver besitzen ― ähnlich wie die Basisverbindung Zinkoxid ― medizinisch und pharmazeutisch interessante Eigenschaften. Die Erfindung betrifft daher auch Artikel (insbesondere pharmazeutische Zubereitungen), die eine antimikrobiell wirksame und/oder eine die Wundheilung fördernde Menge an erfindungsgemäßen Partikeln oder erfindungsgemäßen Pulvern umfassen. Die erfindungsgemäßen Partikel/Pulver lassen sich auch hervorragend in Präparate gegen Zinkmangelerkrankungen einarbeiten, z. B. gegen: Acrodermatits enteropathica (eine erbliche Hautkrankheit), Leberzirrhose, Sichelzellenanämie, Diabetes, rheumatische Athritis, Ateriosklerose, offene Beine. Solche Präparate sind vorzugsweise zur inneren Anwendung vorgesehen. Zinkpräparate mit einem Anteil an den erfindungsgemäßen Partikeln/Pulvern sind auch einsetzbar bei: Verbrennungen, Wunden, diversen Hauterkrankungen, und Infektionen. Dabei entspricht die Formulierung eines erfindungsgemäßen Präparats regelmäßig der Formulierung entsprechender pharmazeutischer Präparate auf Basis von Zinkoxid.

Die Erfindung betrifft auch ein Verfahren zur Herstellung der erfindungsgemäßen Partikel oder Pulver. Das erfindungsgemäße Verfahren umfasst dabei die folgenden Schritte:
Gemeinsames Erhitzen von ZnO und ZnS, vorzugsweise in Gegenwart eines Halogens oder einer Halogenverbindung. Ein altematives erfindungsgemäßes Verfahren zur Herstellung der erfindungsgemäßen Partikel oder Pulver umfasst die folgenden Schritte:
   - Erzeugen einer wässrigen Suspension von Zinkhydroxid,
   - Umsetzen des Zinkhydroxids mit Sulfidionen, so dass sich ein Niederschlag bildet,
   - Tempern des Niederschlags.
Dabei wird in beiden alternativen erfindungsgemäßen Verfahren durch das "gemeinsame Erhitzen" bzw. "Tempern" eine Festkörperreaktion bewirkt, die letztlich zu den erfindungsgemäßen Partikeln oder Pulvern führt.

Die Erfindung betrifft auch die Verwendung der erfindungsgemäßen Partikel oder Pulver als Pigmente, Erfindungsgemäße Partikel/Pulver mit einem Anteil an Mn-Atomen im Bereich 0,001 < y < 0,2 sind besonders wertvolle Pigment-Partikel bzw. -Pulver, denn der Einbau von Mangan-Atomen führt zu einer intensiv weinroten Farbe.

Schließlich betrifft die Erfindung auch ein Verfahren zur Absorption von UV-Strahlung mit folgendem Schritt: Bestrahlen eines erfindungsgemäßen Partikels oder Pulvers mit UV-Strahlung,

Weitere bevorzugte Ausgestaltungen der vorliegenden Erfindung ergeben sich aus den beigefügten Patentansprüchen und den nachfolgenden Beispielen:

### Beispiel 1:

Herstellung eines Pulvers einer Verbindung der Formel ZnO₁₋ₓSₓ (x ≤0,05).

In separaten Untersuchungen wurden Gemenge folgender Zusammensetzungen hergestellt:
21,78mmol ZnO / 0,22 mmol ZnS
21,56 mmol ZnO / 0,44 mmol ZnS
21,34 mmol ZnO / 0,66 mmol ZnS
21,12 mmol ZnO / 0,88 mmol ZnS
20,90 mmol ZnO / 1,10 mmol ZnS

Die Komgrößen der eingesetzten Ausgangsstoffe lagen jeweils im Bereich von 1-10 µm.

Jedes Gemenge umfasst somit insgesamt 22 mol Zn. Der rechnerische Wert für x variierte zwischen 0,01 und 0,05.

Die Gemenge wurden jeweils in Quarzglasbehälter überführt, diese evakuiert und anschließend mit wenig Brom befüllt, verschlossen und für mindestens 5 Tage bei einer Temperatur von 950 °C gehalten. Es wurden einphasige, hellgelbe Pulver einer Verbindung der Formel ZnO₁₋ₓSₓ gebildet, wobei die Partikel des gebildeten Pulvers eine Komgröße im Bereich von 1-10 µm besaßen.

Anmerkung: In zusätzlichen Untersuchungen wurde festgestellt, dass bei einem rechnerischen Wert von x über 0,05 zusätzlich zur Phase ZnO₁₋ₓSₓ eine zweite Phase mit einem hohen Schwefelanteil gebildet wurde.

### Beispiel 2:

Herstellung von Kristallen (großen kristallinen Partikeln) einer Verbindung der Formel ZnO₁₋ₓSₓ durch Abscheidung aus der Gasphase:

Es wurden Gemenge aus ZnO und ZnS hergestellt, zum Beispiel ein Gemenge bestehend aus 1701 mg (20,9 mmol) Zinkoxid und 107 mg (1,1 mmol) Zinksulfid.

Die Gemenge wurden in einer Quarzglasampulle (Innertdurchmesser 10 mm; Länge 190 mm) in einem waagerecht stehenden Zweizonenofen einem Temperaturgefälle von zum Beispiel 1000 °C → 900 °C ausgesetzt. Etwas Brom (alternativ eine entsprechende Menge eines anderen Halogens oder einer Halogenverbindung) wurde als Transportmittel zugesetzt; der Bromdruck (Anfangsdruck) lag bei 0,5 bar. Es wurden mehrere hellgrünliche, verwachsene ZnO₁₋ₓSₓ-Kristalle mit einer Größe von mehreren Millimetern erhalten.

Die Gitterparameter der ZnO₁₋ₓSₓ-Phasen wurden röntgenographisch ermittelt; sie unterscheiden sich von denen des reinen ZnO nur geringfügig: *a* = 325,251(17) pm, *c* = 520,906(6) pm, *V* = 47,742(4) . 10⁶ pm³. ZnO (zum Vergleich): *a* = 325,156(5) pm, *c* = 520,906(6) pm, *V* = 47,6951(14) 10⁶ pm³.

### Beispiel 3:

Herstellung eines Pulvers einer Verbindung der Formel ZnO₁₋ₓSₓ durch Fällung aus Lösung:
Erster Schritt:
Eine wässrige Zinksalzlösung, zum Beispiel eine gesättigte ZnSO₄-Lösung, wurde mit Natriumhydroxid versetzt, so dass Zinkhydroxid als Niederschlag ausfiel. Das Zinkhydroxid wurde abfiltriert.
Zweiter Schritt:
Der Niederschlag wurde mit destilliertem Wasser bis zur Neutralität gewaschen.
Dritter Schritt:

Der gewaschene Niederschlag wurde in Wasser suspendiert und in die resultierende Suspension für 15 Minuten H₂S-Gas eingeleitet. Altemativ wurde in anderen Verfahrensgestaltungen eine wässrige H₂S-Lösung oder eine andere Zubereitung, die Sulfidionen bereitstellt, zugesetzt.

Der resultierende Niederschlag wurde filtriert und anschließend getempert, bevorzugt für zunächst 12 Stunden bei 500 °C und dann für weitere 12 Stunden bei 800 °C.

Das Produkt war ein hellgelbes Pulver einer Verbindung der Formel ZnO₁₋ₓSₓ.

### Analytische Methoden:

In eigenen Untersuchungen wurden die folgenden analytischen Methoden zur Bestimmung der Zusammensetzung, zur Bestätigung der Einphasigkeit erfindungsgemäßer Produkte und zur Bestimmung der Komgrößenverteilung eingesetzt:
Methoden zur Bestimmung der Zusammensetzung:
   Energiedispersive Röntgenspektroskopie: EDX (EDAX, SUTW-Detektor, Röntgenanalytik Messtechnik GmbH. ProgrammVision 32). Wellenlängendispersive Röntgenspektroskopie: WDX. (Cameca)
   Methode zur Bestätigung der Einphasigkeit:
   Röntgenpulverdiffraktometrie(Stadi P mit PSD, Stoe, Damnstadt, Cu-K_{α-}Strahlung, 40 kV, 30 mA. Auswertungssoftware: WinXPow, Fa. Stoe).
   Methode zur Bestimmung der Korngrößenverteilung:
   Rasterelektronenmikroskopie: REM (FEI-Philips, XL 30, W-Kathode). Dynamische Lichtstreuung (Zetasizer Nano Series, Malvem Instruments Ltd. 2003)

### UV/Vis-Spektren von Einkristallen von Verbindungen der Formeln ZnO, ZnS und ZnO₁₋ₓSₓ:

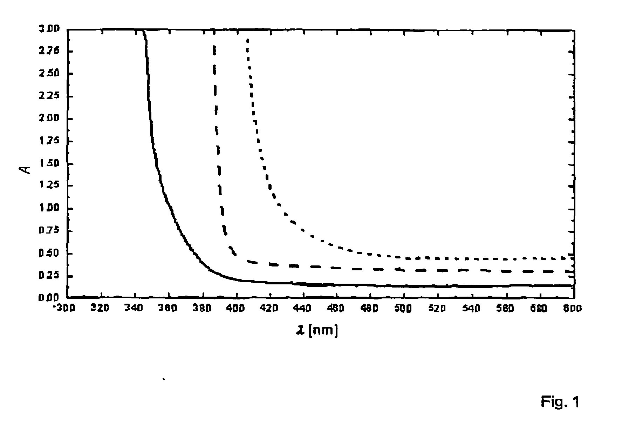

Es wurden UV/Vis-Spektren von Einkristallen von Verbindungen der Formeln ZnO. ZnS und ZnO₁₋ₓSₓ aufgenommen und in der beigefügten Figur 1 graphisch dargestellt. In Figur 1 bedeutet:
- λ [nm]:: Wellenlänge
- A:: Absorbanz: A = log(I₀/I), wobei I₀ und I die Intensität des einfiallenden und transmittierten Lichts sind
- durchgezogene Linie:: Graph für ZnS
- gestrichelte Linie:: Graph für ZnO
- fein gestrichelte Linie:: Graph für ZnO₁₋ₓSₓ (x = 0,05)

In Figur 1 wurde für ZnO (gestrichelte Linie) eine y-Verschiebung um 0,5 Einheiten und für ZnO₁₋ₓSₓ (fein gestrichelte Linie) eine y-Verschiebung um 0,3 Einheiten vorgenommen.

## Patentansprüche

1. Partikel oder Pulver einer Verbindung der Formel Zn_{1-y}M_{y}O₁₋ₓSₓ, in der
x einen Wert im Bereich von 0,01 bis 0,08 besitzt,
M ein zweiwertiges Metall ausgewählt aus der Gruppe bestehend aus: Cr, Mn, Fe, Co, Ni, Cu, Cd und Mg bedeutet und
y einen Wert im Bereich von 0 bis 0,2 besitzt, wobei
die Verbindung Wurtzit-Struktur besitzt.

2. Partikel oder Pulver nach einem der vorangehenden Ansprüche, wobei y den Wert 0 oder einen Wert im Bereich von 0 bis 0,0002 besitzt.

3. Partikel oder Pulver nach einem der vorangehenden Ansprüche, wobei x einen Wert im Bereich von 0,01 bis 0,05 besitzt.

4. Partikel oder Pulver nach einem der vorangehenden Ansprüche, wobei das Partikel bzw. das Pulver einphasig ist.

5. Partikel oder Pulver nach einem der vorangehenden Ansprüche, wobei das Partikel eine Partikelgröße bzw. das Pulver eine mittlere Komgröße von 300 nm oder mehr besitzt.

6. Partikel oder Pulver nach Anspruch 5, wobei das Partikel eine Partikelgröße bzw, das Pulver eine mittlere Komgröße im Bereich von 1 bis 500 µm besitzt, vorzugsweise im Bereich von 1 bis 10 µm.

7. Artikel, umfassend Partikel oder Pulver nach einem der vorangehenden Ansprüche.

8. Artikel nach Anspruch 7, wobei der Artikel die Partikel oder das Pulver im Bereich seiner Oberfläche umfasst, so dass die Partikel oder das Pulver bei Gebrauch des Artikels eine UV-absorbierende Wirkung entfalten können.

9. Artikel nach einem der Ansprüche 7 oder 8, wobei der Artikel eine kosmetische oder pharmazeutische Zubereitung, ein Kunststoff, eine Farbe oder ein Lack ist.

10. Artikel nach einem der Ansprüche 7 bis 9, wobei der Artikel eine antimikrobiell wirksame und/oder eine die Wundheilung fördernde Menge an Partikeln oder Pulver nach einem der Ansprüche 1 bis 6 umfasst.

11. Verwendung eines Partikels oder Pulvers nach einem der Ansprüche 1 bis 6 als UV-Absorptionsmittel.

12. Verfahren zur Herstellung von Partikeln oder Pulvern nach einem der vorangehenden Ansprüche 1 bis 6, mit folgenden Schritten:
Gemeinsames Erhitzen von ZnO und ZnS, vorzugsweise in Gegenwart eines Halogens oder einer Halogenverbindung.

13. Verfahren zur Herstellung von Partikeln oder Pulvern nach einem der vorangehenden Ansprüche 1 bis 6, mit folgenden Schritten:
- Erzeugen einer wässrigen Suspension von Zinkhydroxid,
- Umsetzen des Zinkhydroxids mit Sulfidionen, so dass sich ein Niederschlag bildet,
- Tempern des Niederschlags.

14. Verfahren zur Absorption von UV-Strahlung, mit folgendem Schritt:
- Bestrahlen eines Partikels oder Pulvers nach einem der vorangehenden Ansprüche 1 bis 6 mit UV-Strahlung.

## Claims

1. Particle or powder of a compound of the formula Zn_{1-y}M_{y}O₁₋ₓSₓ, in which
x has a value in the range from 0.01 to 0.08,
M represents a divalent metal selected from the group consisting of: Cr, Mn, Fe, Co, Ni, Cu, Cd and Mg and
y has a value in the range from 0 to 0.2, wherein
the compound has a wurtzite structure.

2. Particle or powder according to one of the preceding claims, wherein y has a value of 0 or a value in the range from 0 to 0.0002.

3. Particle or powder according to one of the preceding claims, wherein x has a value in the range from 0.01 to 0.05.

4. Particle or powder according to one of the preceding claims, wherein the particle or powder is single-phase.

5. Particle or powder according to one of the preceding claims, wherein the particle has a particle size or the powder has a mean grain size of 300 nm or more.

6. Particle or powder according to claim 5, wherein the particle has a particle size or the powder has a mean grain size in the range from 1 to 500 µm, preferably in the range from 1 to 10 µm.

7. Article comprising particles or powder according to one of the preceding claims.

8. Article according to claim 7, wherein the article comprises the particles or powder in the region of its surface, so that the particles or powder are able to develop a UV-absorbing action when the article is used.

9. Article according to one of claims 7 or 8, wherein the article is a cosmetic or pharmaceutical preparation, a plastics material, a paint or a surface coating.

10. Article according to one of claims 7 to 9, wherein the article comprises particles or powder according to one of claims 1 to 6 in an antimicrobially effective amount and/or in an amount that promotes the healing of wounds.

11. Use of a particle or powder according to one of claims 1 to 6 as a UV absorber.

12. Process for the preparation of particles or powders according to one of the preceding claims 1 to 6, comprising the following steps:
heating ZnO and ZnS together, preferably in the presence of a halogen or halogen compound.

13. Process for the preparation of particles or powders according to one of the preceding claims 1 to 6, comprising the following steps:
- producing an aqueous suspension of zinc hydroxide,
- reacting the zinc hydroxide with sulfide ions so that a precipitate forms,
- subjecting the precipitate to heat.

14. Method of absorbing UV radiation, comprising the following step:
- irradiating a particle or powder according to one of the preceding claims 1 to 6 with UV radiation.

## Revendications

1. Particule ou poudre d'un composé de formule Zn_{1-y}M_{y}O₁₋ₓSₓ, dans lequel:
x vaut de 0,01 à 0,08 ;
M représente un métal bivalent choisi dans le groupe consistant en : Cr, Mn, Fe, Co, Ni, Cu, Cd et Mg ; et
y a une valeur dans la gamme de 0 à 0,2 ; et
le composé a une structure de type wurtzite.

2. Particule ou poudre selon l'une des revendications précédentes, dans laquelle y vaut 0 ou a une valeur dans la gamme de 0 à 0,0002.

3. Particule ou poudre selon l'une des revendications précédentes, dans laquelle x a une valeur dans la gamme de 0,01 à 0,05.

4. Particule ou poudre selon l'une des revendications précédentes, dans laquelle la particule ou la poudre est monophasée.

5. Particule ou poudre selon l'une des revendications précédentes, dans laquelle la particule présente une taille ou la poudre présente une taille moyenne de grain de 300 nm ou plus.

6. Particule ou poudre selon la revendication 5, dans laquelle la particule présente une taille ou la poudre présente une taille moyenne de grain de 1 à 500 µm, de préférence de 1 à 10 µm.

7. Article comprenant une particule ou une poudre selon l'une des revendications précédentes.

8. Article selon la revendication 7, dans lequel l'article comprend la particule ou la poudre au niveau de sa surface, de telle sorte que les particules ou la poudre peuvent développer une action d'absorption UV quand l'article est utilisé.

9. Article selon la revendication 7 ou 8, dans lequel l'article est une préparation cosmétique ou pharmaceutique, une matière plastique, une peinture ou un revêtement.

10. Article selon l'une des revendications 7 à 9, dans lequel l'article comprend des particules ou la poudre selon l'une des revendications 1 à 6 dans une quantité efficace anti-microbiologique et/ou dans une quantité qui favorise la cicatrisation des blessures.

11. Utilisation d'une particule ou d'une poudre selon l'une des revendications 1 à 6 en tant que moyen d'absorption UV.

12. Procédé de préparation de particules ou de poudres selon l'une des revendications précédentes 1 à 6, comprenant les étapes suivantes :
- chauffage en commun de ZnO et ZnS, de préférence en présence d'un halogène ou d'un composé halogéné.

13. Procédé de préparation de particules ou de poudres selon l'une des revendications précédentes 1 à 6, comprenant les étapes suivantes :
- préparation d'une suspension aqueuse d'hydroxyde de zinc ;
- mise en contact de l'hydroxyde de zinc avec des ions sulfures, de telle sorte qu'un précipité se forme ;
- recuit du précipité.

14. Procédé d'absorption de rayonnement UV, comprenant l'étape suivante :
- exposer une particule ou une poudre selon l'une des revendications précédentes 1 à 6 à un rayonnement UV.
